# FASCICULE DE BREVET EUROPEEN

(11) **EP 4 025 133 B1**
(45) Date de publication et mention de la délivrance du brevet: **01.11.2023**
(21) Numéro de dépôt: 20781390.8
(22) Date de dépôt: 03.09.2020
(51) Int. Cl.: A61B 10/00

(54) **DISPOSITIF DE TEST DE LIQUIDE BIOLOGIQUE, EN PARTICULIER DE TEST SALIVAIRE**
TESTVORRICHTUNG FÜR BIOLOGISCHE FLÜSSIGKEITEN, INSBESONDERE SPEICHELTESTVORRICHTUNG
BIOLOGICAL FLUID TEST DEVICE, IN PARTICULAR A SALIVA TEST DEVICE

(30) Priorité: 03.09.2019 FR 1909663
(43) Date de publication de la demande: 13.07.2022
(73) Titulaire: Toda Groupe, 67000 Strasbourg (FR)
(72) Inventeur: BERROS, Yossi, 67000 STRASBOURG (FR)
(74) Mandataire: Touroude & Associates
(86) Numéro de dépôt international: PCT/FR2020/000234
(87) Numéro de publication internationale: WO 2021/044086

(56) Documents cités:
- EP-A2- 1 351 055
- WO-A2-02/097389
- DE-U1- 8 805 565
- US-A1- 2005 220 677
- US-A1- 2006 246 574
- US-A1- 2019 150 836

## Description

La présente invention a trait au domaine des tests de liquides biologiques, en particulier des tests salivaires, pour déceler la présence de certaines substances telles que des drogues, virus, bactéries...

Actuellement sur le marché, il est nécessaire, après la réalisation d'un test drogue qui va s'avérer positif, de confirmer les résultats obtenus par une solution de contre-expertise.

Un dispositif tel que défini dans le préambule de la revendication 1 est divulgué dans le document WO 02/097389 A2.

Il sera dans ce cas nécessaire de prélever à nouveau de la salive dans la bouche de la personne testée (sujet) à l'aide d'un second système de prélèvement afin de le faire parvenir pour contrôler les résultats.

Or d'une part le fait de faire un second prélèvement n'est pas aisé pour le patient et pour le manipulateur et d'autre part lors du second prélèvement, la qualité du prélèvement peut présenter une certaine détérioration due à de nombreux facteurs et notamment au fait que le patient n'est pas disposé à ce que ce prélèvement soit exploitable.

Un objectif de la présente invention est de proposer des moyens de test de liquide biologique limitant les aléas liés à un second prélèvement de contre-expertise, notamment le fait que le nouveau prélèvement n'est pas aisé, le risque de détérioration, le risque de non-collaboration du sujet.

Pour atteindre cet objectif, l'invention propose un dispositif de test de liquide biologique, tel que défini dans les revendications.

Selon un premier aspect, le dispositif comprend, ou est associé à, au moins un moyen pour retirer le collecteur et le réserver à des fins de contre-expertise.

Avantageusement, l'invention implique une solution de collection simplifiée et de conservation de prélèvement salivaire pour réaliser une contre-expertise.

Selon d'autres aspects pris isolément ou combinés selon toutes les combinaisons techniquement réalisables :
- le moyen de contrôle de quantité suffisante comprend une ligne de contrôle ; et/ou
- le moyen de contrôle de quantité suffisante comprend un indicateur ; et/ou
- le moyen pour afficher un résultat de test comprend une fenêtre sur le dispositif, associée à au moins un moyen de révélation du dispositif ; et/ou
- le moyen pour retirer le collecteur comprend une pince ; et/ou
- le moyen pour retirer le collecteur comprend un système d'éjection automatique ; et/ou
- le moyen pour retirer le collecteur comprend une partie sécable du collecteur ; et/ou
- le moyen pour réserver le collecteur comprend une solution de conservation spécifique et/ou un récipient spécifique
- le moyen pour réserver le collecteur comprend un récipient comprenant une solution de conservation.

L'invention porte en outre sur un kit de test de liquide biologique, en particulier de test salivaire, le kit comprenant :
- un dispositif de test selon l'invention, et
- au moins un moyen pour retirer le collecteur et/ou pour le réserver à des fins de contre-expertise, en particulier un récipient spécifique et/ou une solution de conservation spécifique ou des moyens pour réaliser la solution de conservation spécifique.

Un autre objet de l'invention concerne une méthode de réalisation de test de liquide biologique, en particulier de test salivaire, et de contre-expertise si le test est positif, la méthode comprenant les étapes pour :
- effectuer un test salivaire au moyen d'un dispositif de test selon l' invention, ou d'un kit selon l'invention,
- si le test est positif, retirer le collecteur sous forme de membrane, et
- réserver le collecteur sous forme de membrane à des fins de contre-expertise.

Plus généralement, l'invention concerne en outre une méthode de réalisation de test salivaire, et de contre-expertise si le test salivaire est positif, la méthode comprenant les étapes pour :
- effectuer un test salivaire au moyen d'un dispositif de test salivaire comprenant un collecteur de prélèvement sous forme de membrane, qui comprend un moyen de contrôle de quantité suffisante, et un moyen pour afficher, sur le dispositif, un résultat de test salivaire,
- si le test est positif, retirer le collecteur sous forme de membrane, et
- réserver le collecteur sous forme de membrane à des fins de contre-expertise.

L'invention sera davantage détaillée par la description de modes de réalisation non-limitatifs, et sur la base des figures annexées illustrant des variantes de l'invention, dans lesquelles :

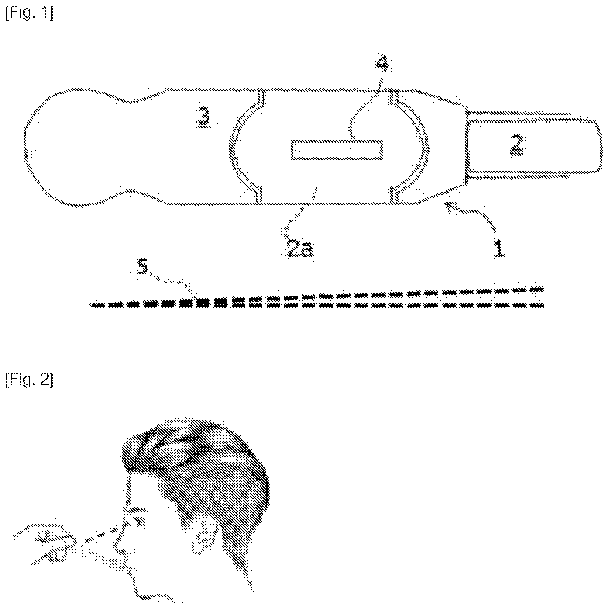

[Fig. 1] est une vue de dessus d'un dispositif de test salivaire selon un mode de réalisation préféré de l'invention, associé à un premier exemple de moyen pour retirer le collecteur ;
[Fig. 2] illustre un prélèvement salivaire au moyen du dispositif de la figure 1 ;
[Fig. 3] est une vue en plan d'un récipient de conservation comprenant un collecteur d'un dispositif selon la figure 1 ;
[Fig. 4] illustre un deuxième exemple de moyen d'éjection du collecteur d'un dispositif selon un mode de réalisation ; et
[Fig. 5] est un schéma de la méthode de réalisation du test et la conservation pour contre-expertise.

L'invention concerne les tests de liquides biologiques pour déceler la présence de certaines substances telles que des drogues, virus, bactéries...

Ces tests sont de préférence configurés pour être réalisés sur des sujets humains. Il s'agit ici de tests salivaires, mais des tests sur d'autres liquides biologiques, tels que les urines, peuvent être envisagés sur le même principe.

L'invention une application dans les procédures de test salivaire où une contre-expertise est requise, sans avoir besoin de refaire un second prélèvement, évitant ainsi les aléas associés (nouveau prélèvement non aisé, risque de détérioration/non-collaboration...).

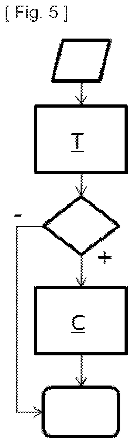

Ce type d'application peut être illustré par une partie de la figure 5. En substance, un test salivaire est effectué sur un sujet dans une première étape T. Si le test est positif, une contre-expertise (C) doit être réalisée, par exemple par des analyses plus poussées.

Ainsi, l'invention concerne un dispositif de test de liquide biologique 1. Il s'agit en particulier d'un dispositif de test salivaire.

Le dispositif comprend un collecteur de prélèvement 2 sous forme de membrane. Le collecteur 2 peut avoir une forme sensiblement plane. Cela permet de limiter l'encombrement du dispositif au niveau du collecteur, et en facilite le stockage.

Le collecteur 2 comprend un matériau absorbant permettant de retenir le liquide biologique prélevé et de le faire migrer par exemple par capillarité.

Le collecteur est associé à un corps 3 du dispositif comprenant une section de migration du liquide biologique 2a, en particulier de la salive. Il s'agit par exemple d'un boîtier renfermant au moins une bandelette de migration du liquide biologique. Le collecteur 2 est par exemple en contact avec la bandelette de migration 2a du corps 3 du dispositif 1. On peut envisager un collecteur 2 en une seule pièce avec la bandelette 2a.

Le dispositif 1 comprend en outre un moyen de contrôle de quantité suffisante. Il s'agit en particulier d'un ou plusieurs éléments du dispositif, plus particulièrement du corps 3 du dispositif, permettant de signaler à l'utilisateur qu'une quantité suffisante de liquide a été prélevée.

Le collecteur 2 est donc configuré pour faire migrer le liquide biologique jusqu'au moyen de contrôle.

Selon une variante, le moyen de contrôle de quantité suffisante comprend une ligne de contrôle. Il s'agit par exemple d'un système adsorbé réagissant au contact du liquide biologique pour révéler une ligne colorée, par exemple en fonction du pH, d'un réactif spécifique du liquide biologique tel que l'amylase ou d'un système d'immunoglobulines recombinantes.

Alternativement ou en combinaison, le moyen de contrôle de quantité suffisante comprend un indicateur. Il s'agit par exemple d'un indicateur coloré sensible au pH du liquide biologique ou à un réactif spécifique du liquide biologique.

Le dispositif 1 comprend en outre un moyen pour afficher un résultat de test 4 pour au moins une substance recherchée. Il s'agit en particulier d'une fenêtre 4 sur le dispositif 1, en particulier sur le boitier et en regard de la bandelette de migration 2a.

La fenêtre 4 est associée à au moins un moyen de révélation du dispositif 1, en particulier sur la bandelette de migration.

Il s'agit par exemple d'un système adsorbé réagissant au contact du de la substance recherchée pour révéler une ligne colorée, par exemple sur la base d'une réaction avec une fonction réactive spécifique de la substance recherchée ou d'un système d'immunoglobulines recombinantes.

L'affichage permet d'avoir un premier résultat de test sans avoir à retirer le collecteur 2 pour effectuer des analyses poussées, et le faire si le premier résultat est positif.

Selon l'invention, le dispositif 1 est associé à au moins un moyen pour retirer le collecteur 2 afin de le réserver à des fins de contre-expertise.

Dans une variante, il s'agit par exemple d'une pince 5 permettant de saisir le collecteur 2 et de le retirer par exemple en tirant vers l'extérieur.

Dans une autre variante, il s'agit d'un moyen de coupe tel que des ciseaux ou un scalpel, de préférence associé à une section sécable du collecteur.

Alternativement ou en combinaison, selon l'invention, le dispositif comprend un moyen pour retirer le collecteur afin de le réserver à des fins de contre-expertise. Il s'agit par exemple d'un système d'éjection automatique du collecteur 5a, 5b. Ce type de système est configuré pour faire ressortir le collecteur.

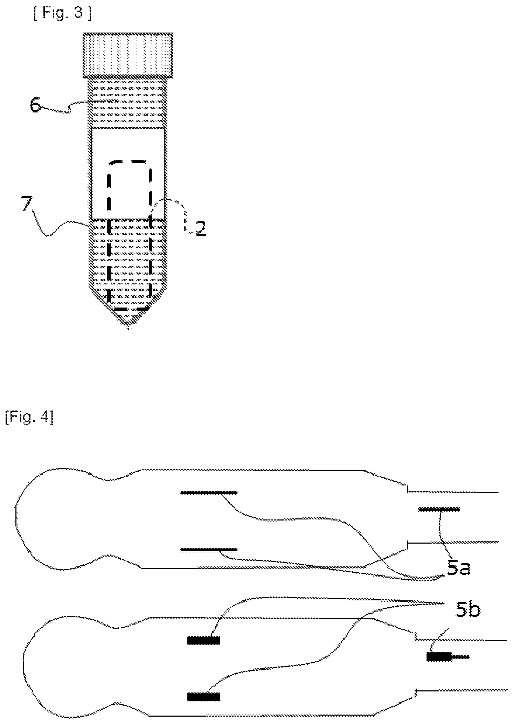

La figure 4 illustre un moyen d'éjection automatique. La figure du haut illustre la face arrière du dispositif qui comprend des percements et fentes 5a pour emboîter un socle d'éjection. La figure du bas illustre le socle 5b venant s'emboiter dans l'arrière du dispositif 1 dans les percements et fentes 5a pour éjecter le collecteur par coulissement.

Le collecteur 2 ainsi retiré, est ensuite réservé par exemple dans un milieu adéquat. Il s'agit par exemple d'une solution tampon 6 spécifique permettant d'éviter la détérioration de l'échantillon. La constitution de cette solution dépend du liquide biologique en cause et de la substance recherchée.

La solution de conservation 6 peut être optimisée pour favoriser l'extraction des composés recherchés.

Le collecteur 2 peut être réservé dans un récipient spécifique 7, par exemple ne laissant pas passer la lumière. Cela permet d'éviter la détérioration de composés recherchés sensibles à la lumière.

Avantageusement, l'invention implique une solution de collection simplifiée et de conservation de prélèvement salivaire pour réaliser une contre-expertise. Le collecteur 2 peut être préservé de manière adéquate de sorte à éviter les détériorations du prélèvement, et optimiser les extractions. Une bonne extraction des composés recherchés permet d'améliorer les tests poussés ultérieurs.

L'invention évite de devoir presser un collecteur en appliquant des forces importantes, car cela ne permet pas d'extraire une quantité satisfaisante de composés recherchés piégés dans le collecteur 2.

L'invention porte en outre sur un kit de test de liquide biologique correspondant. Il s'agit, en particulier d'un kit de test salivaire.

Le kit comprend un dispositif de test 1 tel que décrit précédemment, et au moins un moyen tel que décrit précédemment pour retirer le collecteur 2 et le réserver à des fins de contre-expertise.

Dans une variante, il s'agit d'une solution de conservation spécifique ou des moyens pour réaliser la solution de conservation spécifique 6. Par exemple, le kit inclut une composition à solubiliser avec de l'eau distillée.

Dans une variante, on peut prévoir un récipient spécifique 7 par exemple empêchant le passage de lumière.

L'invention porte en outre sur une méthode de réalisation de test de liquide biologique correspondante. Il s'agit en particulier d'une méthode de réalisation de test salivaire, et de contre-expertise si le test est positif.

La méthode comprend une étape (T) pour effectuer un test salivaire au moyen d'un dispositif de test tel que décrit précédemment, ou d'un kit tel que décrit précédemment.

La suite de la méthode dépend du résultat du test, positif (+) ou négatif (-).

Si le test est négatif, la méthode prend fin.

Si le test est positif, la méthode comprend en outre une étape de retirer le collecteur sous forme de membrane 2, en particulier avec un moyen tel que décrit précédemment.

La méthode comprend en outre une étape (C) de réserver le collecteur sous forme de membrane 2 à des fins de contre-expertise, en particulier avec un des moyens décrit précédemment.

Si une contre-expertise est à réaliser, le récipient 7 peut être utilisé pour réaliser des tests plus poussés.

## Revendications

1. Dispositif de test de liquide biologique (1), en particulier de test salivaire, le dispositif comprenant :
- un collecteur de prélèvement (2) sous forme de membrane,
- un moyen de contrôle de quantité suffisante, le collecteur étant configuré pour faire migrer un liquide biologique jusqu'au moyen de contrôle,
- un moyen de test pour au moins une substance recherchée,
- un moyen pour afficher, sur le dispositif, un résultat de test pour au moins une substance recherchée,
**caractérisé en ce que** le dispositif (1) comprend, ou est associé à, au moins un moyen (5 ; 5a, 5b, 6) pour retirer le collecteur et le réserver à des fins d'analyse plus poussée de contre-expertise.

2. Dispositif de test selon la revendication précédente, **caractérisé en ce que** le moyen de test comprend une bandelette de migration (2a) en contact ou en une seule pièce avec le collecteur (2).

3. Dispositif de test selon l'une des revendications précédentes, **caractérisé en ce que** le moyen de contrôle de quantité suffisante comprend un indicateur et/ou une ligne de contrôle.

4. Dispositif de test selon l'une des revendications précédentes, **caractérisé en ce que** le moyen pour afficher un résultat de test comprend une fenêtre (4) sur le dispositif, associée à au moins un moyen de révélation du dispositif (1).

5. Dispositif de test selon l'une des revendications précédentes, **caractérisé en ce que** le moyen pour retirer le collecteur comprend une pince (5).

6. Dispositif de test selon l'une des revendications précédentes, **caractérisé en ce que** le moyen pour retirer le collecteur comprend un système d'éjection automatique (5a, 5b).

7. Dispositif de test selon l'une des revendications précédentes, **caractérisé en ce que** le moyen pour retirer le collecteur comprend une partie sécable du collecteur (2).

8. Dispositif de test selon l'une des revendications précédentes, **caractérisé en ce que** le moyen pour réserver le collecteur comprend une solution de conservation spécifique (6) formant un milieu adéquat dépendant du liquide biologique en cause et d'une substance recherchée et/ou un récipient (7) spécifique d'un composé recherché.

9. Kit de test de liquide biologique, en particulier de test salivaire, le kit comprenant :
- un dispositif de test (1) selon l'une des revendications précédentes, et
- au moins un moyen pour retirer le collecteur et/ou pour le réserver à des fins de contre-expertise (6, 7), en particulier un récipient spécifique (7) et/ou une solution de conservation spécifique (6) ou des moyens pour réaliser la solution de conservation spécifique (6).

10. Méthode de réalisation de test de liquide biologique, en particulier de test salivaire, et de contre-expertise si le test est positif, la méthode comprenant les étapes pour :
- effectuer un test salivaire au moyen d'un dispositif de test (1) selon l'une des revendications 1 à 8, ou d'un kit selon la revendication précédente,
- si le test est positif, retirer le collecteur sous forme de membrane (2), et
- réserver le collecteur (2) sous forme de membrane à des fins de contre-expertise.

## Patentansprüche

1. Vorrichtung zum Testen, insbesondere Speicheltesten, einer biologischen Flüssigkeit (1), die Vorrichtung umfassend:
- einen Probensammler (2) in Form einer Membran,
- ein Mittel zum Kontrollieren einer ausreichenden Menge, wobei der Sammler konfiguriert ist, um eine biologische Flüssigkeit zu dem Kontrollmittel zu migrieren,
- ein Testmittel für mindestens eine gesuchte Substanz,
- ein Mittel zum Anzeigen eines Testergebnisses für mindestens eine gesuchte Substanz auf der Vorrichtung,
**dadurch gekennzeichnet, dass** die Vorrichtung (1) mindestens ein Mittel (5; 5a, 5b, 6) umfasst oder diesem zugeordnet ist, um den Sammler zu entfernen und diesen zu Zwecken der ausführlicheren Gegengutachtenanalyse aufzubewahren.

2. Testvorrichtung nach dem vorstehenden Anspruch,
**dadurch gekennzeichnet, dass** das Testmittel einen Migrationsstreifen (2a) in Kontakt oder einstückig mit dem Sammler (2) umfasst.

3. Testvorrichtung nach einem der vorstehenden Ansprüche,
**dadurch gekennzeichnet, dass** das Mittel zum Kontrollieren einer ausreichenden Menge einen Indikator und/oder eine Kontrolllinie umfasst.

4. Testvorrichtung nach einem der vorstehenden Ansprüche,
**dadurch gekennzeichnet, dass** das Mittel zum Anzeigen eines Testergebnisses ein Fenster (4) auf der Vorrichtung umfasst, das mindestens einem Erkennungsmittel der Vorrichtung (1) zugeordnet ist.

5. Testvorrichtung nach einem der vorstehenden Ansprüche,
**dadurch gekennzeichnet, dass** das Mittel zum Entfernen des Sammlers eine Pinzette (5) umfasst.

6. Testvorrichtung nach einem der vorstehenden Ansprüche,
**dadurch gekennzeichnet, dass** das Mittel zum Entfernen des Sammlers ein automatisches Ausstoßsystem (5a, 5b) umfasst.

7. Testvorrichtung nach einem der vorstehenden Ansprüche,
**dadurch gekennzeichnet, dass** das Mittel zum Entfernen des Sammlers einen teilbaren Teil des Sammlers (2) umfasst.

8. Testvorrichtung nach einem der vorstehenden Ansprüche,
**dadurch gekennzeichnet, dass** das Mittel zum Aufbewahren des Sammlers eine spezifische Konservierungslösung (6), die ein geeignetes Medium ausbildet, abhängig von der betreffenden biologischen Flüssigkeit und einer gesuchten Substanz, und/oder einen spezifischen Behälter (7) einer gesuchten Zusammensetzung.

9. Kit zum Testen, insbesondere Speicheltesten, einer biologischen Flüssigkeit, das Kit umfassend:
- eine Testvorrichtung (1) nach einem der vorstehenden Ansprüche, und
- mindestens ein Mittel zum Entfernen des Sammlers und/oder zum Aufbewahren dessen zu Zwecken des Gegengutachtens (6, 7), insbesondere einen spezifischen Behälter (7) und/oder eine spezifische Konservierungslösung (6) oder Mittel zum Herstellen der spezifischen Konservierungslösung (6).

10. Verfahren zum Durchführen des Tests, insbesondere des Speicheltests, der biologischen Flüssigkeit und des Gegengutachtens, falls der Test positiv ist, das Verfahren umfassend die Schritte zum:
- Ausführen eines Speicheltests mittels einer Testvorrichtung (1) nach einem der Ansprüche 1 bis 8 oder eines Kits nach dem vorstehenden Anspruch,
- falls der Test positiv ist, Entfernen des Sammlers (2) in Form einer Membran, und
- Aufbewahren des Sammlers (2) in Form einer Membran zu Zwecken des Gegengutachtens.

## Claims

1. Biological fluid test device (1), in particular saliva test device, the device comprising:
- a sample collector (2) in the form of a membrane,
- a means for checking whether the quantity is sufficient, the collector being configured to cause a biological fluid to migrate to the checking means,
- a means for testing for at least one target substance,
- a means for displaying, on the device, a test result for at least one target substance,
**characterized in that** the device (1) comprises, or is associated with, at least one means (5; 5a, 5b, 6) for removing the collector and storing it for the purpose of further second-opinion analysis.

2. Test device according to the preceding claim, **characterized in that** the test means comprises a migration strip (2a) which is in contact with or integral with the collector (2).

3. Test device according to one of the preceding claims, **characterized in that** the means for checking whether the quantity is sufficient comprises an indicator and/or a control line.

4. Test device according to one of the preceding claims, **characterized in that** the means for displaying a test result comprises a window (4) on the device, which window is associated with at least one revealing means of the device (1).

5. Test device according to one of the preceding claims, **characterized in that** the means for removing the collector comprises pincers (5).

6. Test device according to one of the preceding claims, **characterized in that** the means for removing the collector comprises an automatic ejection system (5a, 5b).

7. Test device according to one of the preceding claims, **characterized in that** the means for removing the collector comprises a scored part of the collector (2).

8. Test device according to one of the preceding claims, **characterized in that** the means for storing the collector comprises a specific preservation solution (6) forming a suitable medium, depending on the biological fluid in question and a target substance, and/or a specific container (7) of a target compound.

9. Biological fluid test kit, in particular saliva test kit, the kit comprising:
- a test device (1) according to one of the preceding claims, and
- at least one means (6, 7) for removing the collector and/or for storing it for the purpose of a second opinion, in particular a specific container (7) and/or a specific preservation solution (6) or means for producing the specific preservation solution (6).

10. Method for performing a biological fluid test, in particular a saliva test, and for providing a second opinion if the test is positive, the method comprising the steps of:
- performing a saliva test using a test device (1) according to one of claims 1 to 8, or a kit according to the preceding claim,
- if the test is positive, removing the collector (2) in the form of a membrane, and
- storing the collector (2) in the form of a membrane for the purpose of a second opinion.
